# EUROPÄISCHE PATENTSCHRIFT

(11) **EP 0 781 319 B1**
(45) Veröffentlichungstag und Bekanntmachung des Hinweises auf die Patenterteilung: **16.08.2000**
(21) Anmeldenummer: 95932001.1
(22) Anmeldetag: 07.09.1995
(51) Int. Cl.: C11D 1/37, B01F 17/00

(54) **MILDE DETERGENSGEMISCHE**
MILD DETERGENT MIXTURES
MELANGES DETERGENTS DOUX

(30) Priorität: 16.09.1994 DE 4433071
(43) Veröffentlichungstag der Anmeldung: 02.07.1997
(73) Patentinhaber: Cognis Deutschland GmbH, 40589 Düsseldorf (DE)
(72) Erfinder: FABRY, Bernd, D-41352 Korschenbroich (DE); BEHLER, Ansgar, D-46240 Bottrop (DE)
(86) Internationale Anmeldenummer: EP9503505
(87) Internationale Veröffentlichungsnummer: WO9608551

(56) Entgegenhaltungen:
- EP-A- 0 417 619
- WO-A-91/14761
- WO-A-92/21318
- DE-A- 3 637 683
- DE-C- 4 410 000
- FR-A- 936 632
- FR-A- 1 459 789
- FR-A- 1 466 141
- FR-A- 1 500 775
- GB-A- 1 170 092
- DATABASE WPI Week 9001 Derwent Publications Ltd., London, GB; AN 90-004588 & JP,A,01 288 267 (TOKUYAMA SODA KK) , 20.November 1989

## Beschreibung

### Gebiet der Erfindung

Die Erfindung betrifft Detergensgemische mit verbesserter hautkosmetischer Verträglichkeit, enthaltend Monoglycerid(ether)sulfate und ausgewählte Aminosäurederivate, die diese Gemische enthalten sowie die Verwendung der Gemische zur Herstellung von oberflächenaktiven Mitteln.

### Stand der Technik

Monoglyceridsulfate stellen formal Anlagerungsprodukte von Schwefeltrioxid an die primäre Hydroxylgruppe eines Glycerinmonofettsäureesters dar. Technisch gesehen handelt es sich jedoch um komplexe Aniontensidgemische, die üblicherweise durch gleichzeitige Umesterung und Sulfatierung von Gemischen aus Triglyceriden und Glycerin sowie nachfolgender Neutralisation erhalten werden.

Monoglyceridsulfate zeichnen sich durch zufriedenstellende anwendungstechnische Eigenschaften und gute dermatologische Verträglichkeit aus. Übersichten zu Herstellung und Eigenschaften von Monoglyceridsulfaten sind beispielsweise von **A.K. Biswas et al. in J.Am.Oil.Chem.Soc. 37, 171 (1960), R. Chamanial et al. in J.Oil.Technol.Ass.lnd. 41 (1972) und J.K.Jain in Indian J.Pharm.Sci. 41, 181 (1979)** erschienen.

Aus der **US 4554097** (Colgate-Palmolive) sind Detergensgemische bekannt, die neben Kokosmonoglyceridsulfaten Gelatine enthalten.

Für eine Reihe von Anwendungen ist das Schaumvermögen, insbesondere bei Härtebelastung, sowie die hautkosmetische Verträglichkeit der Monoglyceridsulfate sowie der dazu analogen Ethersulfate nicht voll befriedigend.

Die Aufgabe der Erfindung hat demnach darin bestanden, einen Weg zu finden, Performance, und Hautverträglichkeit von Monoglycerid(ether)sulfaten signifikant zu verbessern.

### Beschreibung der Erfindung

Gegenstand der Erfindung sind milde Detergensgemische, enthaltend
(a) Monoglycerid(ether)sulfate der Formel (I), 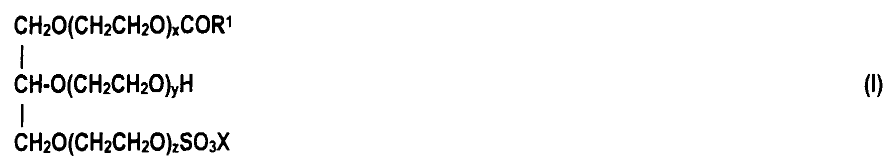 in der R¹CO für einen linearen oder verzweigten Acylrest mit 6 bis 22 Kohlenstoffatomen, x, y und z in Summe für 0 oder für Zahlen von 1 bis 30 und X für ein Alkali- oder Erdalkalimetall steht, und
(b) Aminosäurederivate ausgewählt aus der Gruppe, die gebildet wird von
   (b1) Acylglutamaten der Formel (II), 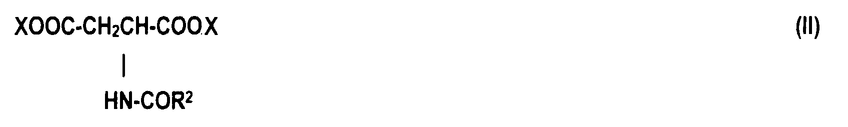 in der R²CO für einen linearen oder verzweigten Acylrest mit 6 bis 22 Kohlenstoffatomen und 0 und/oder 1, 2 oder 3 Doppelbindungen und X für Wasserstoff, ein Alkali- und/oder Erdalkalimetall, Ammonium, Alkylammonium, Alkanolammonium oder Glucammonium steht und/oder
   (b2) Proteinfettsäurekondensate auf Basis von pflanzlichen Proteinen und Fettsäuren mit 12 bis 18 Kohlenstoffatomen.

Überraschenderweise wurde gefunden, daß Abmischungen von Monoglyceridsulfaten bzw. Monoglyceridethersulfaten und den genannten Aminosäurederivaten zu einer in synergistischer Weise verbesserten hautkosmetischen Verträglichkeit bei gesteigertem Schaumvermögen führt.

### Monoglyceride und Monoglyceridethersulfate

Monoglyceridsulfate und Monoglyceridethersulfate stellen bekannte anionische Tenside dar, die nach den einschlägigen Methoden der präparativen organischen Chemie erhalten werden können. Üblicherweise geht man zu ihrer Herstellung von Triglyceriden aus, die gegebenenfalls nach Ethoxylierung zu den Monoglyceriden umgeestert und nachfolgend sulfatiert und neutralisiert werden. Gleichfalls ist es möglich, die Partialglyceride mit geeigneten Sulfatierungsmitteln, vorzugsweise gasförmiges Schwefeltrioxid oder Chlorsulfonsäure umzusetzen [vgl. **WO 92109569**, **WO 92109570**, Henkel]. Die neutralisierten Stoffe können - falls gewünscht - einer Ultrafiltration unterworfen werden, um den Elektrolytgehalt auf ein gewünschtes Maß zu vermindern.

Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukten mit Schwefeltrioxid oder Chlorsulfonsäure in Form ihrer Natriumsalze. Vorzugsweise werden Monoglyceridsulfate der Formel (I) eingesetzt, in der R¹CO für einen linearen Acylrest mit 8 bis 18 Kohlenstoffatomen steht.

### Acylglutamate

Acylglutamate stellen bekannte anionische Tenside dar. Ihre Herstellung erfolgt beispielsweise durch Schotten-Baumann Acylierung von Glutaminsäure mit Fettsäuren, Fettsäureestem oder -chloriden. Verkaufsprodukte sind beispielsweise von der Hoechst AG, Frankfurt/DE oder der Ajinomoto Co. Inc., Tokyo/JP erhältlich. Eine Übersicht zu Herstellung und Eigenschaften der Acylglutamate findet sich von M.Takehara et al. in **J.Am.Oil.Chem.** Soc., **49**, **143 (1972)**.

Glutaminsäure stellt formal eines der Produkte dar, das man bei einer Totalhydrolyse von Proteinen erhalten würde. Tatsächlich führt die Hydrolyse von Proteinen jedoch zu Gemischen von Oligopeptiden, die noch im Mittel 5 bis 20 Aminosäureeinheiten aufweisen. Demzufolge stellen übliche Proteinfettsäurekondensate Acylierungsprodukte von Oligopeptiden dar. Acylglutamate unterscheiden sich von diesen Stoffen dadurch, daß sie gewissermaßen "Monomere" darstellen.

Typische Beispiele für geeignete Acylglutamate, die im Sinne der Erfindung in Betracht kommen, sind Aniontenside, die sich von Fettsäuren mit 6 bis 22, vorzugsweise 12 bis 18 Kohlenstoffatomen ableiten, wie beispielsweise C_{12/14}- bzw. C_{12/18}-Kokosfettsäure, Laurinsäure, Myristinsäure, Palmitinsäure und/oder Stearinsäure. Besonders bevorzugt sind Natrium-N-cocoyl- und Natrium-N-stearoyl-L-glutamat.

### Pflanzliche Proteinhydrolysate

Pflanzliche Proteinhydrolysate stellen Abbauprodukte beispielsweise von Erbsen-, Kartoffel-, Reis-, Mandel- und vorzugsweise Soja- oder Weizenproteinen dar, die durch saure, alkalische und/oder enzymatische Hydrolyse gespalten werden und danach ein durchschnittliches Molekulargewicht im Bereich von 600 bis 4000, vorzugsweise 2000 bis 3500 aufweisen. Obschon Proteinhydrolysate in Ermangelung eines hydrophoben Restes keine Tenside im klassischen Sinne darstellen, finden sie wegen ihrer dispergierenden Eigenschaften vielfach Verwendung von Formulierung oberflächenaktiver Mittel. Übersichten zu Herstellung und Verwendung von Proteinhydrolysaten sind beispielsweise von G.Schuster und A.Domsch in **Seifen Öle Fette Wache, 108**, **177 (1982)** bzw. Cosm.Toil. **99**, **63 (1984)**, von H.W. Steisslinger in **Parf.Kosm. 72, 556 (1991)** und F.Aurich et al.in **Tens.Surf.Det. 29, 389 (1992)** erschienen.

### Proteinfettsäurekondensate

Proteinfettsäurekondensate stellen bekannte Stoffe dar, zu deren Herstellung man vorzugsweise von den oben genannten pflanzlichen Proteinhydrolysaten ausgeht, die einer Schotten-Baumann-Acylierung vorzugsweise unter Einsatz von Fettsäurechloriden unterworfen werden. Übersichten zu diesem Thema sind beispielsweise von M.Naudet in **Bull.Soc.Chim.Fr., 358 (1950)**, G. Schuster et al. in **Parf.Kosm. 45, 337 (1964)** und O.J.Muscio et al. in **J.Am.Oil.Chem.Soc. 59 217 (1982)** erschienen.

Die im Sinne der Erfindung einzusetzenden Proteinhydrolysate stellen formal Acylierungsprodukte von vorzugsweise Weizen- bzw. Sojaproteinhydrolysaten mit aliphatischen Fettsäuren der Formel **(III)** dar,

**R**^{**3**}**CO-OH** **(III)**

in der R³CO für einen aliphatischen Acylrest mit 6 bis 22, vorzugsweise 12 bis 18 Kohlenstoffatomen steht. Wie schon beschrieben, wird der Fettacylrest jedoch nicht über die Fettsäuren, sondern über die Fettsäurechloride in die Kondensate eingeführt. Wenn also im folgenden ausgeführt wird, von welchen Fettsäuren sich die Proteinfettsäurekondensate ableiten können, dann ist damit die Lehre zum technischen Handeln verknüpft, zu ihrer Herstellung die entsprechenden Fettsäurechloride einzusetzen.

Beispiele für Fettsäuren, von denen sich die Proteinfettsäurekondensate formal ableiten können, sind: Capronsäure, Caprylsäure, 2-Ethylhexansäure, Isononansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die beispielsweise durch Druckspaltung von Fetten und Ölen oder Reduktion von Aldehyden aus der Roelen'schen Oxosynthese erhältlich sind.

Die Proteinfettsäurekondensate können in Form ihrer Alkali-, Erdalkali- und/oder Ammoniumsalze, vorzugsweise als Natrium-, Kalium-, Magnesium- und/oder Calciumsalze eingesetzt werden. Bevorzugt ist der Einsatz von Proteinfettsäurekondensaten auf Basis von Weizen- und/oder Sojaprotein bzw. deren Hydrolysaten und Fettsäuren mit 12 bis 18 Kohlenstoffatomen. Weiterhin bevorzugt sind hochacylierte Proteinfettsäurekondensate mit einem Gesamtstickstoffgehalt im Bereich von 1,8 bis 4,1 und vorzugsweise 2,5 bis 3,5.

### Tenside

Die erfindungsgemäßen Detergensgemische können weitere anionische, nichtionische kationische und/oder amphotere Tenside enthalten.

Typisch Beispiele für **anionische Tenside** sind Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Fettsäureamid(ether)sulfate, Mono- und Dialkylsulfosuccinate, Mono- und Dialkylsulfosuccinamate, Sulfotriglyceride, Amidseifen, Alkyloligoglucosidsulfate und Alkyl(ether)phosphate. Sofern die anionischen Tenside Polyglycoletherketten enthalten, können sie eine konventionelle, vorzugsweise jedoch eine eingeengte Homologenverteilung aufweisen.

Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, Alk(en)yloligoglykoside und Fettsäure-N-alkylglucamide. Sofern die nichtionischen Tenside Polyglycoletherketten enthalten, können sie eine konventionelle, vorzugsweise jedoch eine eingeengte Homologenverteilung aufweisen.

Typische Beispiele für **kationische Tenside** sind quartäre Ammoniumverbindungen und Esterquats, insbesondere quaternierte Fettsäuretrialkanolaminester-Salze.

Typische Beispiele für **amphotere bzw. zwitterionische Tenside** sind Alkylbetaine, Alkylamidobetaine, Aminopropionate, Aminoglycinate, Imidazoliniumbetaine und Sulfobetaine.

Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Cosnumer Products", Springer Verlag, Berlin, 1987, S. 54-124** oder **J.Falbe** **(ed.), "Katalysatoren, Tenside und Mineralöladditive", Thieme Verlag, Stuttgart, 1978, S. 123-217** verwiesen.

Die erfindungsgemäßen Detergensgemische können die oben genannten zusätzlichen Tenside in Anteilen von 1 bis 50, vorzugsweise 5 bis 25 Gew.-% - bezogen auf den Feststoffanteil der Gemische - enthalten.

### Gewerbliche Anwendbarkeit

Die erfindungsgemäßen Detergensgemische zeichnen sich durch ein besonders vorteilhaftes Schaumvermögen und eine in synergistischer Weise verbesserte hautkosmetische Verträglichkeit aus, Eigenschaften, die bei der Entwicklung einer Vielzahl von oberflächenaktiven Mitteln von Bedeutung ist:

Weitere Gegenstände der Erfindung betreffen daher oberflächenaktive Mitteln, die einen Gehalt dieser Detergensgemische aufweisen und die im folgenden näher definiert werden:
- **Pulverförmige Universalwaschmittel**, enthaltend 10 bis 30 Gew.-% - bezogen auf das Mittel - der erfindungsgemäßen Detergensgemische sowie übliche Hilfs- und Zusatzstoffe.
- **Flüssige Universalwaschmittel**, enthaltend 10 bis 70 Gew.-% - bezogen auf das Mittel - der erfindungsgemäßen Detergensgemische sowie übliche Hilfs- und Zusatzstoffe.
- **Flüssige Feinwaschmittel**, enthaltend 10 bis 50 Gew.-% - bezogen auf das Mittel - der erfindungsgemäßen Detergensgemische sowie übliche Hilfs- und Zusatzstoffe.
- **Avivagemittel**, enthaltend 10 bis 50 Gew.-% - bezogen auf das Mittel - der erfindungsgemäßen Detergensgemische sowie übliche Hilfs- und Zusatzstoffe.
- **Handgeschirrspülmittel**, enthaltend 10 bis 50 Gew.-% - bezogen auf das Mittel - der erfindungsgemäßen Detergensgemische sowie übliche Hilfs- und Zusatzstoffe.
- **Klarspüler**, enthaltend 10 bis 50 Gew.-% - bezogen auf das Mittel - der erfindungsgemäßen Detergensgemische sowie übliche Hilfs- und Zusatzstoffe.
- **Flüssige Reinigungs- und Desinfektionsmittel**, enthaltend 10 bis 30 Gew.-% - bezogen auf das Mittel - der erfindungsgemäßen Detergensgemische sowie übliche Hilfs- und Zusatzstoffe.
- **Stückseifen vom Kombibar-Typ**, enthaltend 1 bis 2 Gew.-% - bezogen auf das Mittel - der erfindungsgemäßen Detergensgemische sowie übliche Hilfs- und Zusatzstoffe.
- **Syndetseifen**, enthaltend 1 bis 2 Gew.-% - bezogen auf das Mittel - der erfindungsgemäßen Detergensgemische sowie übliche Hilfs- und Zusatzstoffe.
- **Haarshampoos**, enthaltend 10 bis 30 Gew.-% - bezogen auf das Mittel - der erfindungsgemäßen Detergensgemische sowie übliche Hilfs- und Zusatzstoffe.
- **Haarspülungen**, enthaltend 10 bis 30 Gew.-% - bezogen auf das Mittel - der erfindungsgemäßen Detergensgemische sowie übliche Hilfs- und Zusatzstoffe.
- **Haarfärbemittel**, enthaltend 10 bis 30 Gew.-% - bezogen auf das Mittel - der erfindungsgemäßen Detergensgemische sowie übliche Hilfs- und Zusatzstoffe.
- **Haarwellmittel**, enthaltend 10 bis 30 Gew.-% - bezogen auf das Mittel - der erfindungsgemäßen Detergensgemische sowie übliche Hilfs- und Zusatzstoffe.
- **Schaumbäder**, enthaltend 10 bis 30 Gew.-% - bezogen auf das Mittel - der erfindungsgemäßen Detergensgemische sowie übliche Hilfs- und Zusatzstoffe.
- **Textil- und Faserhilfsmittel**, enthaltend 1 bis 30 Gew.-% - bezogen auf das Mittel - der erfindungsgemäßen Detergensgemische sowie übliche Hilfs- und Zusatzstoffe.
- **Lederfettungsmittel**, enthaltend 1 bis 30 Gew.-% - bezogen auf das Mittel - der erfindungsgemäßen Detergensgemische sowie übliche Hilfs- und Zusatzstoffe.
- **Flotationshilfsmittel**, enthaltend 1 bis 30 Gew.-% - bezogen auf das Mittel - der erfindungsgemäßen Detergensgemische sowie übliche Hilfs- und Zusatzstoffe.
- **Hilfsmittel für die Feststoffentwässerung**, enthaltend 1 bis 30 Gew.-% - bezogen auf das Mittel - der erfindungsgemäßen Detergensgemische sowie übliche Hilfs- und Zusatzstoffe.

### Hilfs- und Zusatzstoffe

**Wasch-, Spül-, Reinigungs- und Avivagemittel** auf Basis der erfindungsgemäßen Detergensgemische können - neben den bereits genannten Tenside - als weitere Hilfs- und Zusatzstoffe beispielsweise Builder, Salze, Bleichmittel, Bleichaktivatoren, optische Aufheller, Vergrauungsinhibitoren, Lösungsvermittler und Enzyme enthalten.

Übliche **Builder** sind Natriumaluminiumsilicate (Zeolithe), Phosphate, Phosphonate, Ethylendiamintetraessigsäure, Nitrilotriacetat, Citronensäure und/oder Polycarboxylate.

Als Salze bzw. Stellmittel kommen beispielsweise Natriumsulfat, Natriumcarbonat oder Natriumsilicat (Wasserglas) in Betracht. Als typische Einzelbeispiele für weitere Zusatzstoffe sind Natriumborat, Stärke, Saccharose, Polydextrose, TAED, Stilbenverbidnugnen, Methylcellulose, Toluolsulfonat, Cumolsulfonat, langkettige Seifen, Silicone, Mischether, Lipasen und Proteasen zu nennen.

**Haarshampoos**, **Haarlotionen** oder **Schaumbäder** können als weitere Hilfs- und Zusatzstoffe - neben den bereits genannten Tensiden - **Emulgatoren** wie etwa alkoxylierte Fettalkohole oder Sorbitanester enthalten.

Als **Überfettungsmittel** können Substanzen wie beispielsweise polyethoxylierte Lanolinderivate, Lecithinderivate und Fettsäurealkanolamide verwendet werden, wobei die letzteren gleichzeitig als Schaumstabilisatoren dienen.

Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon sowie Elektrolyte wie Kochsalz und Ammoniumchlorid.

Unter **biogenen Wirkstoffen** sind beispielsweise Pflanzenextrakte und Vitaminkomplexe zu verstehen. Gebräuchliche **Filmbildner** sind beispielsweise Chitosan, mikrokristallines Chitosan, quatemiertes Chitosan, Polyvinylpyrrolidon, Vinylpyrrolidon-Vinyl-acetat-Copolymerisate, Polymere der Acrylsäurereihe, quatemäre Cellulose-Derivate und ähnliche Verbindungen.

Als **Konservierungsmittel** eignen sich beispielsweise Phenoxyethanol, Formaldehydlösung, Parabene, Pentadiol oder Sorbinsäure.

Als **Perlglanzmittel** kommen beispielsweise Glycoldistearinsäureester wie Ethylenglycoldistearat, aber auch Fettsäuremonoglycolester in Betracht.

Als Farbstoffe können die für kosmetische Zwecke geeigneten und zugelassenen Substanzen verwendet werden, wie sie beispielsweise in der Publikation "**Kosmetische Färbemittel" der Farbstoffkommission der Deutschen Forschungsgemeinschaft, veröffentlicht im Verlag Chemie, Weinheim**, **1984, S. 81-106** zusammengestellt sind. Diese Farbstoffe werden üblicherweise in Konzentrationen von 0,001 bis 0,1 Gew.-% , bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen.

Die erfindungsgemäßen Detergensgemische eignen sich zur Herstellung von flüssigen oder festen oberflächenaktiven Mitteln, in denen sie in Mengen von 1 bis 99 und vorzugsweise 10 bis 90 Gew.-% - bezogen auf den Feststoffgehalt der Mittel - enthalten sein können.

### Beispiele

### I. Eingesetzte Tenside

A1) C_{12/18}-Kokosmonoglyceridsulfat-Natriumsalz
B1) N-Laurylglutamat-Natriumsalz
B2) Weizenprotein-Kokosfettsäure Kondensat-Kaliumsalz

### II. Anwendungstechnische Ergebnisse

Das Schaumvermögen wurde nach der DIN-Methode 53 902, Teil 2 (Ross-Miles-Test) durchgeführt. Eingesetzt wurden 1 Gew.-%ige Tensidlösungen in Wasser von 16°d; die Temperatur betrug 20°C. Bestimmt wurden Basisschaum und Schaumvolumen nach 5 min.

Die Bestimmung des Reizpotentials erfolgte gemäß der OECD-Methode No. 404 und der EEC Directive 84/449 EEC, Pt.B.4. Die angegebenen Reizsummenscores wurden aus den nach 24, 48 und 72 Stunden erhaltenen Reizscores gebildet. Dabei wurde der im Vergleichsversuch V1 ermittelte Reizsummenscore für ein 100 %iges C_{12/18}-Kokosfettsäuremonoglyceridsulfat-Natriumsalz zu 100 % gesetzt und die in den übrigen Versuchen erhaltenen Reizsummenscores zu diesem ins Verhältnis gesetzt.

Die Ergebnisse sind in Tabelle 1 zusammengefaßt (Prozentangaben als Gew.-%).

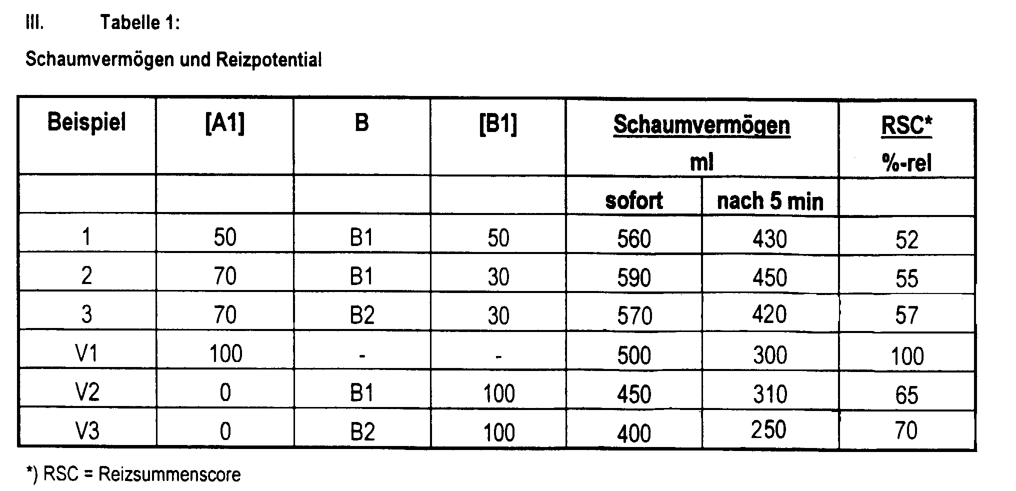

## Patentansprüche

1. Milde Detergensgemische, enthaltend
(a) Monoglycerid(ether)sulfate der Formel **(I)**, 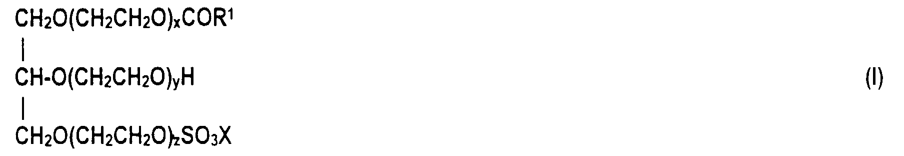 in der R¹CO für einen linearen oder verzweigten Acylrest mit 6 bis 22 Kohlenstoffatomen, x, y und z in Summe für 0 oder für Zahlen von 1 bis 30 und X für ein Alkali- oder Erdalkalimetall steht, und
(b) Aminosäurederivate ausgewählt aus der Gruppe, die gebildet wird von
(b1) Acylglutamaten der Formel **(II)**, 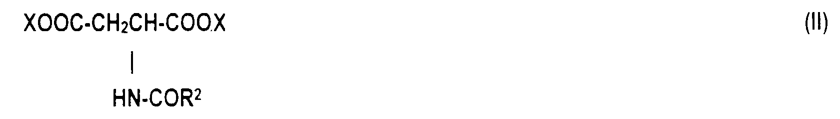 in der R²CO für einen linearen oder verzweigten Acylrest mit 6 bis 22 Kohlenstoffatomen und 0 und/oder 1, 2 oder 3 Doppelbindungen und X für Wasserstoff, ein Alkali- und/oder Erdalkali-metall, Ammonium, Alkylammonium, Alkanolammonium oder Glucammonium steht und/oder
(b2) Proteinfettsäurekondensate auf Basis von pflanzlichen Proteinen und Fettsäuren mit 12 bis 18 Kohlenstoffatomen.

2. Detergensgemische nach Anspruch 1, **dadurch gekennzeichnet**, daß sie Proteinfettsäure-kondensate auf Basis von Weizen- und/oder Sojaproteinhydrolysat und Fettsäuren mit 12 bis 18 Kohlenstoffatomen enthalten.

3. Detergensgemische nach den Ansprüchen 1 und 2, **dadurch gekennzeichnet**, daß sie die Monoglyce-rid(ether)sulfate und die Aminosäurederivate im Gewichtsverhältnis 50 : 50 bis 70 : 30 enthalten.

4. Verwendung der Detergensgemische nach Anspruch 1 zur Herstellung von oberflächenaktiven Mitteln.

## Claims

1. Mild detergent mixtures containing
(a) monoglyceride (ether) sulfates corresponding to formula **(I)**: 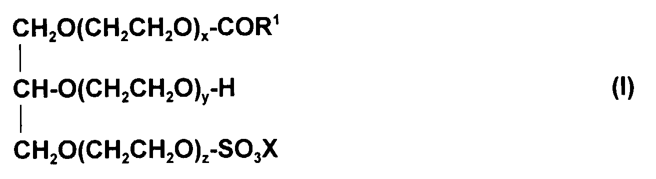 in which R¹CO is a linear or branched acyl group containing 6 to 22 carbon atoms, x, y and z together are 0 or a number of 1 to 30 and X is an alkali metal or alkaline earth metal, and
(b) amino acid derivatives selected from the group consisting of
(b1) acyl glutamates corresponding to formula **(II)**: 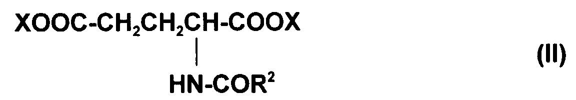 in which R²CO is a linear or branched acyl group containing 6 to 22 carbon atoms and 0 and/or 1, 2 or 3 double bonds and X is hydrogen, an alkali metal and/or alkaline earth metal, ammonium, alkylammonium, alkanolammonium or glucammonium and/or
(b2) protein fatty acid condensates based on vegetable proteins and fatty acids containing 12 to 18 carbon atoms.

2. Detergent mixtures as claimed in claims 1, characterized in that they contain protein fatty acid condensates based on wheat and/or soya protein and fatty acids containing 12 to 18 carbon atoms.

3. Detergent mixtures as claimed in claim 1 and 2, characterized in that they contain the monoglyceride (ether) sulfates and the amino acid derivatives in a ratio by weight of 50:50 to 70:30.

4. Use of detergent mixtures as claimed in claim 1 for the production of surface-active formulations.

## Revendications

1. Mélanges détergents doux, renfermant
a) des (éther)sulfates de monoglycérides de la formule (I), 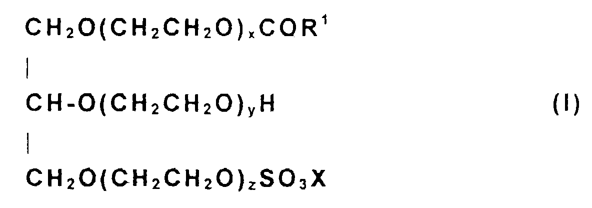 dans laquelle R¹CO représente un radical acyle à chaîne droite ou ramifiée comportant 6 à 22 atomes de carbone, la somme de x, y et z est égale à 0 ou à un nombre de 1 à 30, et X correspond à un métal alcalin ou alcalino-terreux, et
(b) des dérivés d'acides aminés sélectionnés parmi le groupe formé des
(b1) acylglutamates de la formule (II) 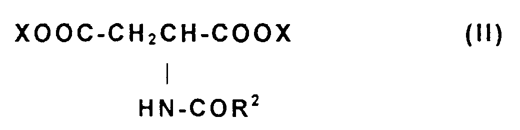 dans laquelle R²CO représente un radical acyle à chaîne droite ou ramifiée comportant 6 à 22 atomes de carbone, et 0 et/ou 1, 2 ou 3 doubles liaisons, et X est l'hydrogène, un métal alcalin et/ou alcalino-terreux, un ammonium, un alkylammonium, un alcanolammonium ou un glucammonium et/ou des
(b2) condensats d'acides gras protéiques à base de protéines végétales et d'acides gras comportant 12 à 18 atomes de carbone.

2. Mélanges détergents doux selon la revendication 1, **caractérisés en ce qu'ils** renferment des condensats d'acides gras protéiques à base d'hydrolysats de protéines de froment et/ou de soja et d'acides gras comportant 12 à 18 atomes de carbone

3. Mélanges détergents doux selon les revendications 1 et 2, **caractérisés en ce qu'ils** renferment les (éther)sulfates de monoglycérides et les dérivés d'acides aminés dans un rapport pondéral de 50:50 à 70:30.

4. L'utilisation des mélanges détergents doux selon la revendication 1 pour la production d'agents tensioactifs.
